# EUROPEAN PATENT APPLICATION

(11) **EP 1 752 434 A1**
(43) Date of publication of application: **14.02.2007**
(21) Application number: 05017341.8
(22) Date of filing: 10.08.2005
(51) Int. Cl.: C07C 2/30, B01J 19/00

(54) **Improved method for preparation of linear alpha-olefins and reactor system therefore**

(71) Applicant: Linde AG, 65189 Wiesbaden (DE); Saudi Basic Industries Corporation, Riyadh 11422 (SA)
(72) Inventor: Fritz, Peter, Dr., 82008 Unterchaching (DE); Bölt, Heinz, 82515 Wolfratshausen (DE); Mosa, Fuad, 11422 Riyadh (SA); Zahoor, Mohammed, Dr., 11422 Riyadh (SA); Aburaqua, Athieh, Dr., 11422 Riyadh (SA)
(74) Representative: Winkler, Andreas Fritz Ernst

(57) **Abstract**

The present invention relates to a method for the preparation of linear alpha-olefins by oligomerization of ethylene in a reactor in the presence of an organic solvent and a homogenous catalyst consisting of a zirconium component and an aluminum component, characterized in that the zirconium component and the aluminum component are premixed in a pre-mixing device, prior to introduction into the reactor, at a temperature range of about 20°C to about 100°C and with a residence time in the pre-mixing device of maximum 75 hours.

## Description

The present invention relates to a method for the preparation of linear alpha-olefins by oligomerization of ethylene in a reactor in the presence of an organic solvent and a homogenous catalyst consisting of a zirconium component and an aluminum component, and a reactor system therefore.

Methods for the preparation of linear alpha-olefins by oligomerization of ethylene are widely known in the art. For example, DE 43 38 414 C1 discloses a method which is carried out in an empty tubular reactor having inlets and outlets for ethylene, oligomerization products and solvent-catalyst mixture. The catalytic components are dissolved in the solvent and are introduced into the reactor at a pressure of 31 bar and at a temperature of 40°C. However, at a pressure of 31 bar an oligomerization reaction can already take place which may result in plugging of pipes for delivering the catalyst-solvent mixture.

Additionally, methods are known in the art wherein the catalyst components are separately introduced into the oligomerization reactor. Thus, accurate dosing of the components is required to achieve desired catalyst concentration and ratio of the components. Further, the formation of the active catalyst has to be performed only in the oligomerization reactor.

It is therefore an object of the present invention to provide a method for the preparation of linear alpha-olefins which overcomes the disadvantages of the prior art. Especially, a method shall be provided having improved productivity, improved homogeneity of the liquid reaction phase and simplified operation and reactor stability.

It is a further object of the present invention to provide a reactor system for carrying out the inventive method.

This object is achieved by a method characterized in that the zirconium component and the aluminum component are pre-mixed in a pre-mixing device, prior to introduction into the reactor, at a temperature range of about 20°C to about 100°C and with a residence time in the pre-mixing device of maximum 75 hours.

Preferably, the pre-mixing is carried out continuously in a separate vessel, static mixer, continuously stirred tank or batchwise.

In one embodiment, the solvent is toluene.

Additionally, the zirconium component may have the formula ZrCl₄₋ₘXₘ, wherein X = OCOR or OSO₃R' with R and R' being independently alkyl, alkene and phenyl, and wherein 0<m<4.

Preferably, the organoaluminum component is Al(C₂H₅)₃, Al₂Cl₃(C₂H₅)₃ or AlCl(C₂H₅)₂ or mixtures thereof.

More preferred, the pre-mixing device is maintained under ethylene pressure of less than 5 bar, preferably under atmospheric pressure.

Further, pre-mixing is carried out preferably at a temperature of about 50-70°C and with a residence time in the pre-mixing device of about 15 to about 120 minutes, preferably about 30 to about 60 minutes.

Finally, the second object is achieved by a reactor system for the preparation of linear alpha-olefins by oligomerization of ethylene comprising a reactor and a pre-mixing device connected thereto to pre-mix catalytic components in a solvent, prior to introduction into the reactor.

Surprisingly, it was found that the inventive method overcomes the drawbacks of the prior art. Especially, an improved productivity of the oligomerization reactor is obtained, as the preformation of the catalyst is done outside of the reactor. Further, an improved homogeneity of the liquid reaction phase is obtained. Further, start-up and operation of the method is simplified, since the catalyst ratio cannot become wrong within the oligomerization reactor. Also the reactor stability is improved, since a reactor runaway due to over-dosing of one catalyst component is avoided.

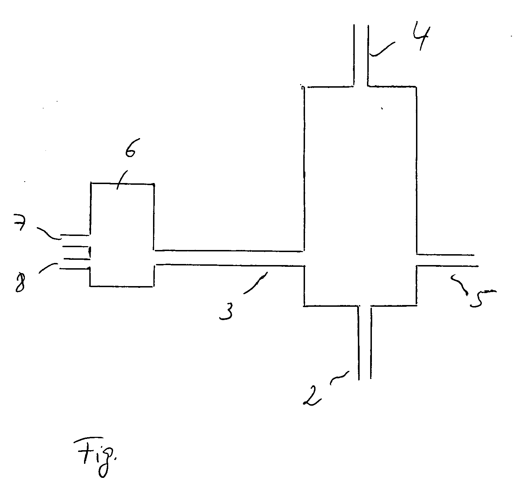

Additional advantages and features of the inventive method are further illustrated with reference to the following examples and the accompanying drawing, wherein the figure schematically illustrates a method according to the present invention.

According to the inventive method, the oligomerization is carried out in an oligomerization reactor 1 having an inlet 2 for introduction of ethylene into the reactor 1, an inlet 3 for introducing a solvent-catalyst mixture, an outlet 4 for removing a mixture of ethylene and light alpha-olefins, and an outlet 5 for removing a liquid mixture consisting of solvent, catalyst, dissolved ethylene and linear alpha-olefins. The inlet 3 is fed with the solvent-catalyst mixture from a pre-mixing device 6 having two inlet-lines 7, 8 for separately introducing the catalyst components (zirconium component and aluminum component) into the pre-mixing device 6. The rate and amount of each catalyst component may be conveniently controlled. The pre-mixing device 6 may be a separate vessel, a static mixer or a continuously stirred tank. The pre-mixing may be carried out continuously or batchwise. Further, the pre-mixing preferably performed at a temperature of about 60°C, wherein the catalytic components have a preferred residence time in the pre-mixing device 6 of about 30 to about 60 minutes. Of course, the catalytic components fed into the pre-mixing device are dissolved in the solvent, preferably toluene.

It is preferred that the pre-mixing device is maintained under an ethylene pressure of less than 5 bar to avoid an oligomerization reaction therein.

### Examples

All materials used for the catalyst were prepared and handled in a glass box filled with nitrogen with less than 1 ppm oxygen and water.

The amount of solvent (toluene), catalyst (ZrCl₄₋ₘXₘ), cocatalyst (EASC) and process parameters are given below.
Toluene = 250 ml
Zr-loading = 0.25 mmol
ZrX₄ concentration in toluene = 1 mmol/l
Al/Zr molar ratio = 35
EASC = ethyl aluminum sesqui chloride
temperature = 80°C
C₂ pressure = 30 bar
reaction time = 1 hour
agitator speed = 800 rpm

In a first group of examples, the catalyst was pre-mixed in a 250 ml round bottom glass flask in a glove box and was stored therein at 20°C from 18-72 hours and then charged into a 2-liter bench scale reactor under nitrogen, whereupon an oligomerization was started at a temperature of 80°C and at an ethylene pressure of 30 bar.

In a second group of examples, fresh catalyst was prepared in a glove box at 20°C and then the catalyst was charged into a 2 liter bench scale reactor maintaining the reactor temperature between 40°C-60°C for 15-120 minutes. Ethylene was introduced into the reactor after the predetermined premixing time until the desired 30 bar pressure was obtained and the reaction temperature was held at 80°C.

The catalytic activity was calculated as the amount of ethylene consumed in one hour. After one hour, the reaction was stopped. A sample of liquid LAO product was collected in a glass veil and analyzed by gas chromatography (GC) at room temperature.

The results of the experiments are given in table 1 below, wherein examples 2 to 5 illustrate the first groups of examples, and examples 6-9 are for the second group. Example 1 of table 1 is an oligomerization run utilizing a standard LAO catalyst without premixing of the catalytic components. The data of examples 2 to 5 indicate that catalyst aging with time (18-72 hours) has increased the catalyst activity, LAO yield and selectivity. It was found that the pre-mixed catalyst was quite stable during this period as compared to standard fresh catalyst.

For examples 6-9 (second group of examples) it was observed that the pre-mixed catalyst was quite stable at various temperatures and aging times as compared to the standard fresh LAO catalyst.

The data show that the catalyst activity, LAO yield and selectivity are increased by more than 10 percent by catalyst aging in the reactor at 60°C for 30-60 minutes (examples 7 and 8 of table 1). The LAO purity was also slightly improved.

In example 10 of table 1, the catalyst was pre-mixed and stored for 30 hours at 20°C in the glove box and was then charged into the reactor, whereupon the reactor temperature was maintained at 50°C for 30 minutes. After that by introduction of ethylene the reaction was started to be conducted at a temperature of 80°C. The results of example 10 show that the catalyst activity, LAO yield and selectivity were slightly increased.

In all experiments described in table 1 no formulation of polymer was observed in the reactor during oligomerisation of ethylene to linear alpha-olefins.

**Table 1. Effect of Catalyst Aging/Pre-mixing on Catalyst Activity and LAO Selectivity**

| | | | | | | | | | | | | | | | | |
|---|---|---|---|---|---|---|---|---|---|---|---|---|---|---|---|---|
| T = 80°C P = 30 Bar Zr = 0.25 mmol Al/Zr = 35 Time = 1 hr ZrX4 conc. = 1 mmol/lt. S = 800 RPM | | | | | | | | | | | | | | | | |
| A - Premixed Cat. Stored at 20°C B - Temp. in Reactor C - Storing Time in Reactor FCCR = Fresh Cat. Charged in Reactor | | | | | | | | | | | | | | | | |

| Expt. No. | A (Hrs) | B (°C) | C (Min.) | C2 (Lts) | Yield (Gm LAO) | Yield (GM LAO / g Zr | LAO Selectivity (wt%) (P = Purity %) | | | | | | | | C12--C18 | C20+ |
|---|---|---|---|---|---|---|---|---|---|---|---|---|---|---|---|---|
| | | | | | | | C4 | P | C6 | P | C8 | P | C10 | P | | |
| 1 | Std. | LAO | Cat. | 220 | 254 | 11154 | 11 | 99,1 | 32 | 96,2 | 22 | 95,9 | 15 | 88,2 | 17 | 3 |
| 2 | 18 | - | - | 235 | 272 | 11915 | 13 | 99 | 34 | 96,2 | 23 | 96 | 14 | 88,1 | 12 | 4 |
| 3 | 24 | - | - | 243 | 281 | 12321 | 14 | 99,2 | 36 | 96,3 | 22 | 95,9 | 13 | 88,2 | 13 | 2 |
| 4 | 48 | - | - | 251 | 290 | 12726 | 15 | 99,1 | 36 | 96,4 | 23 | 96 | 12 | 88,5 | 11 | 3 |
| 5 | 72 | - | - | 257 | 297 | 13030 | 14 | 99,1 | 35 | 96,3 | 22 | 95,9 | 13 | 88,7 | 14 | 2 |
| 6 | FCCR | 40 | 15 | 245 | 283 | 12422 | 14 | 99 | 38 | 96,6 | 24 | 96 | 12 | 89,7 | 10 | 2 |
| 7 | FCCR | 60 | 30 | 255 | 295 | 12929 | 15 | 99,2 | 39 | 97,1 | 24 | 96,1 | 11 | 89,5 | 10 | 1 |
| 8 | FCCR | 60 | 60 | 261 | 302 | 13233 | 14 | 99,1 | 38 | 97 | 23 | 96 | 11 | 89,4 | 13 | 1 |
| 9 | FCCR | 50 | 120 | 266 | 307 | 13487 | 13 | 99 | 36 | 96,5 | 23 | 95,9 | 13 | 89,2 | 13 | 2 |
| 10 | 30 | 50 | 30 | 251 | 290 | 12726 | 13 | 99,1 | 37 | 96,7 | 24 | 96 | 12 | 89,4 | 12 | 2 |

The features disclosed in the foregoing description, in the claims and in the drawing may, both separately and in any combination thereof, be material for realizing the invention in diverse forms thereof.

## Claims

1. Method for the preparation of linear alpha-olefins by oligomerization of ethylene in a reactor in the presence of an organic solvent and a homogenous catalyst consisting of a zirconium component and an aluminum component, **characterized in that** the zirconium component and the aluminum component are pre-mixed in a pre-mixing device, prior to introduction into the reactor, at a temperature range of about 20°C to about 100°C and with a residence time in the pre-mixing device of maximum 75 hours.

2. Method according to claim 1, wherein the pre-mixing is carried out continuously in a separate vessel, static mixer, continuously stirred tank or batchwise.

3. Method according to claim 1 or 2, wherein the solvent is toluene.

4. Method according to any of the preceding claims, wherein the zirconium component has the formula ZrCl₄₋ₘXₘ, wherein X = OCOR or OSO₃R' with R and R' being independently alkyl, alkene and phenyl, and wherein 0<m<4.

5. Method according to any of the preceding claims, wherein the organoaluminum component is Al(C₂H₅)₃, Al₂Cl₃(C₂H₅)₃ or AlCl(C₂H₅)₂ or mixtures thereof

6. Method according to any of the preceding claims, wherein the pre-mixing device is maintained under ethylene pressure of less than 5 bar, preferably under atmospheric pressure.

7. Method according to any of the preceding claims, wherein pre-mixing is carried out at a temperature of about 50-70°C and with a residence time in the pre-mixing device of about 15 to about 120 minutes, preferably about 30 to about 60 minutes.

8. Reactor system for the preparation of linear alpha-olefins by oligomerization of ethylene comprising a reactor and a pre-mixing device connected thereto to pre-mix catalytic components in a solvent, prior to introduction into the reactor.

## Amended claims

### Amended claims in accordance with Rule 86(2) EPC.

**1.** Method for the preparation of linear alpha-olefins by oligomerization of ethylene in a reactor in the presence of an organic solvent and a homogenous catalyst consisting of a zirconium component and an aluminum component, **characterized in that** the zirconium component and the aluminum component are pre-mixed in a pre-mixing device, prior to introduction into the reactor, at a temperature range of 20°C to 100°C and with a residence time in the pre-mixing device of maximum 75 hours.

**2.** Method according to claim 1, wherein the pre-mixing is carried out continuously in a separate vessel, static mixer, continuously stirred tank or batchwise.

**3.** Method according to claim 1 or 2, wherein the solvent is toluene.

**4.** Method according to any of the preceding claims, wherein the zirconium component has the formula ZrCl₄₋ₘXₘ, wherein X = OCOR or OSO₃R' with R and R' being independently alkyl, alkene and phenyl, and wherein 0<m<4.

**5.** Method according to any of the preceding claims, wherein the organoaluminum component is Al(C₂H₅)₃, Al₂Cl₃(C₂H₅)₃ or AlCl(C₂H₅)₂ or mixtures thereof

**6.** Method according to any of the preceding claims, wherein the pre-mixing device is maintained under ethylene pressure of less than 5 bar, preferably under atmospheric pressure.

**7.** Method according to any of the preceding claims, wherein pre-mixing is carried out at a temperature of about 50-70°C and with a residence time in the pre-mixing device of 15 to 120 minutes, preferably 30 to 60 minutes.

**8.** Reactor system for the preparation of linear alpha-olefins by oligomerization of ethylene comprising a reactor and a pre-mixing device connected thereto to pre-mix catalytic components in a solvent, prior to introduction into the reactor.
